# EUROPÄISCHE PATENTSCHRIFT

(11) **EP 1 112 258 B1**
(45) Veröffentlichungstag und Bekanntmachung des Hinweises auf die Patenterteilung: **04.02.2004**
(21) Anmeldenummer: 99944633.9
(22) Anmeldetag: 09.09.1999
(51) Int. Cl.: C07D 251/26, G03C 1/30

(54) **VERFAHREN ZUR HERSTELLUNG EINER STABILISIERTEN WÄSSRIGEN ALKALIMETALL-2-HYDROXY-4,6-DICHLOR-S-TRIAZIN-LÖSUNG UND DEREN VERWENDUNG**
METHOD FOR PRODUCING A STABILIZED AQUEOUS ALKALI METAL-2-HYDROXY-4,6-DICHLORO-S-TRIAZINE SOLUTION AND THE USE THEREOF
PROCEDE DE PREPARATION D'UNE SOLUTION DE METAL ALCALIN-2-HYDROXY-4,6-DICHLORO-S-TRIAZINE AQUEUSE STABILISEE ET SON UTILISATION

(30) Priorität: 10.09.1998 DE 19841383
(43) Veröffentlichungstag der Anmeldung: 04.07.2001
(73) Patentinhaber: Degussa AG, 83308 Trostberg (DE)
(72) Erfinder: NEUMANN, Thomas, D-83308 Trostberg (DE)
(74) Vertreter: Weiss, Wolfgang, Dipl.-Chem. Dr.
(86) Internationale Anmeldenummer: PCT/EP1999/006671
(87) Internationale Veröffentlichungsnummer: WO 2000/015619

(56) Entgegenhaltungen:
- DE-A- 2 820 108
- DE-A- 2 910 726
- CHEMICAL ABSTRACTS, vol. 102, no. 4, 28. Januar 1985 (1985-01-28) Columbus, Ohio, US; abstract no. 36640, ARIENT, JOSEF ET AL: "Aqueous solution of alkali salts of 2-hydroxy-4,6-dichloro-1,3,5-triazine" XP002123477 & CS 223 478 B (CZECH.) 28. Oktober 1983 (1983-10-28)
- CHEMICAL ABSTRACTS, vol. 100, no. 12, 19. März 1984 (1984-03-19) Columbus, Ohio, US; abstract no. 94472, SHAKIROV, R. Z. ET AL: "Hardener based on monosodium salt of 2,4-dichloro-6-hydroxy-1,3,5-triazin" XP002123478 & SU 1 051 082 A (KAZAN SCIENTIFIC-RESEARCH, TECHNOLOGICAL, AND DESIGN INSTITUTE OF THE) 30. Oktober 1983 (1983-10-30)
- CHEMICAL ABSTRACTS, vol. 102, no. 2, 14. Januar 1985 (1985-01-14) Columbus, Ohio, US; abstract no. 14994, KONISHIROKU PHOTO INDUSTRY CO., LTD., JAPAN: "Preparation of photographic gelatin hardening agent" XP002123479 & JP 59 106474 A (KONISHIROKU PHOTO INDUSTRY CO., LTD., JAPAN) 20. Juni 1984 (1984-06-20)

## Beschreibung

Die vorliegende Erfindung beansprucht ein Verfahren zur Herstellung einer stabilisierten wäßrigen Alkalimetall-2-hydroxy-4,6-dichlor-s-triazin- (AHDT) Lösung sowie deren Verwendung.

Die Alkali- und Erdalkalisalze des 2-Hydroxy-4,6-dichlor-s-triazins, vor allem dessen Natriumsalz (NHDT) sind bevorzugte Härtungsverbindungen für gelatinehaltige Materialien, die insbesondere zu photographischen Zwecken eingesetzt werden. Dementsprechend sind auch zahlreiche Verfahren zur Herstellung dieser Triazin-Derivate, aber auch diese enthaltende Härtungslösungen bekannt.

So beschreibt beispielsweise CS 223,478 die Hydrolyse von Cyanurchlorid in NaOH, KOH oder Na₂CO₃ und die anschließende Pufferung mit H₃PO₄, H₃BO₃ oder Na₂B₄O₇ zur Herstellung eines Gelatinehärters. In SU 1,051,082 wird ein Herstellungsverfahren beschrieben, bei dem Cyanursäure in Gegenwart eines Puffersalzes (wie etwa NaBO₂ oder Na₂HPO₄), Phosphat und Lauge umgesetzt wird, um einen photographischen Härter herzustellen. Bei einem weiteren Verfahren (JP 59,106,474) wird Cyanurchlorid mit Hydrogencarbonat umgesetzt und die Härterlösung mit einem Phosphorsäurepuffer versetzt.

Problematisch ist dabei jedoch nach wie vor die Stabilität der Härtungslösungen, da sowohl während der Synthese und Lagerung der mit Carbonaten versetzten Härtungslösungen als auch vielfach während deren Gebrauch eine Kohlendioxidentwicklung abläuft, was mit einer gleichzeitigen Hydrolyse der Triazine zu den entsprechenden Monochlorverbindungen einhergeht. Störend ist diese Kohlendioxidentwicklung vor allem auch deshalb, weil das entstehende Gas Blasen in den mit der Härtungslösung hergestellten photographischen Schichten erzeugt.

Zur Überwindung dieser Schwierigkeiten wurde in der Vergangenheit vorgeschlagen, die wäßrigen Härtungslösungen mit einem wasserlöslichen Borat zu puffern (DE-OS 28 20 108), wobei der Puffereffekt gemäß DE-OS 29 10 726 mit Hilfe eines Alkalimetallhydroxids und ggf. durch Zusatz von wasserlöslichen und unter den Reaktionsbedingungen inerten organischen Lösemitteln, wie z. B. Aceton verstärkt werden soll; die Menge an eingesetztem Alkaliborat beträgt in diesem Fall nur 0,02 bis 2 mol je mol AHDT.

Wie die Praxis zeigt, vermögen aber weder die alleinige Pufferung mit Boraten, noch die zusätzliche Verwendung von Alkalimetallhydroxiden das Problem der CO₂-Entwicklung zu lösen. Nachteilig ist bei den bekannten Syntheseverfahren auch die sehr langsame Bildung des jeweiligen Alkalimetall-2-hydroxy-4,6-dichlor-s-triazins (AHDT).

Es hat sich deshalb die Aufgabe gestellt, ein Verfahren zur Herstellung einer stabilisierten wäßrigen Alkalimetall-2-hydroxy-4,6-dichlor-s-triazin-Lösung bereitzustellen, bei der vor allem die unerwünschte CO₂-Entwicklung sowohl bei der Synthesereaktion als auch während der Lagerung und der Verwendung der fertigen AHDT-Lösung nicht mehr auftritt. Darüber hinaus sollte das Herstellungsverfahren einfach und mit einem geringen Zeitaufwand durchzuführen sein.

Gelöst wurde diese Aufgabe dadurch, daß
(a) eine wäßrige Alkalihydrogencarbonat- und gegebenenfalls Acetonlösung vorgelegt wird, dann
(b) Cyanurchlorid und eine Alkalilauge unter solchen Bedingungen zudosiert werden, daß eine Temperatur von 25°C nicht überschritten und ein pH-Wert von 5,5 nicht unterschritten wird,
(c) danach ein Alkalisalz einer organischen Säure zugesetzt wird,
(d) abschließend ein Stabilisator ausgewählt aus einem Alkaliborat und/oder einem Alkali(hydrogen)phosphat und/oder einem Alkalihexacyanoferrat (II) zugegeben wird, und
(e) gegebenenfalls der pH-Wert mit Säure auf den gewünschten Wert eingestellt wird.

In einer bevorzugten Ausführungsform wird das Verfahren so durchgeführt, daß bei Temperaturen zwischen 5 und 40°C
(a) eine wäßrige 0,1 bis 1,0 Gew.-%ige Alkalihydrogencarbonat- und gegebenenfalls eine 0,5 bis 5 Gew.-%ige Acetonlösung vorgelegt wird, dann
(b) 8 bis 16 Gew.-% Cyanurchlorid bezogen auf die Lösung aus (a) zugesetzt werden, wobei der erhaltenen Suspension während der Cyanurchlorid-Zugabe bei Temperaturen von maximal 25°C 1 Equivalent bezogen auf Cyanurchlorid einer 25 bis 50 Gew.-%igen Alkalilauge so zugegeben wird, daß der pH des Reaktionsgemisches einen Wert von 5,5 nicht unterschreitet,
(c) danach während einer Rührzeit von 2 bis 30 Stunden 0,1 bis 1,5 Gew.-‰ eines Alkalilaurylsulfats bezogen auf die Gesamtlösung zugesetzt werden,
(d) abschließend 0,3 bis 1,2 Gew.-% bezogen auf die Gesamtlösung eines Alkaliborats und/oder Dialkalihydrogenphosphats und/oder Kaliumhexacyanoferrat (II) zugegeben werden, und
(e) ggf. der pH-Wert mit einer Säure auf 8 bis 9,5 eingestellt wird.

Überraschenderweise wurde gefunden, daß sich sehr geringe Acetonmengen oder selbst die völlige Abwesenheit von Aceton überhaupt nicht nachteilig auf den Reaktionsablauf oder die Reaktionszeit auswirken, was aufgrund der bekannten Vorurteile - bspw. aus der DE-OS 29 10 726 - überhaupt nicht zu erwarten war. Darüber hinaus erlaubt vor allem der Einsatz des Alkalihydrogencarbonats eine breite Variierung der Verfahrensführung, wobei zu jedem Zeitpunkt eine quantitative Abpufferung des theoretisch entstehenden Kohlendioxids möglich ist.

Aufgrund des Einsatzes von Alkalihydrogencarbonat ist nun entsprechend dem erfindungsgemäßen Verfahren tatsächlich ein breiter Temperaturbereich von z.B. 5 bis 40°C möglich, wobei allerdings Temperaturen zwischen 10 und 20°C zu bevorzugen sind. Diese relativ niedrigen Temperaturen wirken sich jedoch überhaupt nicht nachteilig auf die Reaktionsgeschwindigkeit des gesamten Verfahrens aus.

Für das vorliegende Verfahren werden Natrium und Kalium als Alkalimetall bevorzugt, deren Einsatz sich als überaus vorteilhaft erwiesen hat. Dabei kann das jeweils eingesetzte Alkalimetall in allen Verfahrensschritten gleich sein, wodurch sich der Vorteil ergibt, daß man entweder das reine Natrium- oder das reine Kalium-2-hydroxy-4,6-dichlor-s-triazin erhält; es sind aber auch Mischungen dieser Produkte möglich.

Das jeweilige Alkalihydrogencarbonat kann dazu entweder als wäßrige Lösung vorgelegt werden, wobei eine 0,5 bis 0,8 Gew.-%ige Lösung zu bevorzugen ist. Die im Verfahrensschritt (a) gegebenenfalls vorliegende wäßrige Acetonlösung soll gemäß vorliegender Erfindung vorzugsweise 1,0 bis 2,0 Gew.-%ig sein. Möglich ist aber auch die Vorlage einer Mischung bestehend aus Alkalihydrogencarbonat und Aceton in beliebigen Verhältnissen, allerdings unter Beachtung der beanspruchten Mengen.

In Abhängigkeit vom jeweils eingesetzten Alkalihydrogencarbonat kann im Verfahrensschritt (b) die Alkalilauge gewählt werden: wird im Verfahrensschritt (a) und/oder (d) NaHCO₃ eingesetzt, so wird vorzugsweise NaOH zudosiert, wird KHCO₃ eingesetzt, wird im Verfahrensschritt (b) vorzugsweise Kalilauge zugegeben.

Insgesamt ist das vorliegende Verfahren somit völlig unkritisch durchzuführen. Es empfiehlt sich allerdings zur Optimierung des Reaktionsablaufes im Verfahrensschritt (b) eine Cyanurchloridmenge einzusetzen, die vorzugsweise bei 10 bis 12 Gew.-% bezogen auf die Lösung aus (a) liegen sollte. Ebenfalls günstig im Sinn einer raschen Reaktionsfolge ist im Verfahrensschritt (c) eine bevorzugte Rührzeit von 3 bis 6 Stunden.

Wie auch die Alkalilauge im Verfahrensschritt (b) so kann auch das jeweilige Alkalisalz, vorzugsweise das Alkalisalz einer C₁₀-C₂₀ Organoschwefelsäure, besonders bevorzugt Alkalilaurylsulfat, im Verfahrensschritt (c) gezielt dem jeweiligen Alkalihydrogencarbonat angepaßt werden, um nicht zuletzt so ein reines Produkt zu gewährleisten.

Wie bereits dargelegt, bestand das vorrangige Ziel bei der Entwicklung des vorliegenden Verfahrens darin, die unerwünschte CO₂-Bildung zu unterdrücken. U. a. gelingt dies durch die Zugabe von Stabilisator- bzw. Puffersubstanzen im Schritt (d) des erfindungsgemäßen Verfahrens, wobei die Alkaliborate Na₂B₄O₇ oder K₂B₄O₇, deren jeweilige Penta- oder Decahydrate oder deren Mischungen zu bevorzugen sind; als Vertreter der ebenfalls im Sinne der Erfindung geeigneten Alkali(hydrogen)phosphate sind Trialkaliphosphate, Alkalidihydrogenphosphate und insbesondere Dialkalihydrogen-phosphate wie das Dinatrium- und das Dikaliumhydrogenphosphat ggf. wieder in Abhängigkeit vom jeweils gewählten Alkalihydrogencarbonat zu bevorzugen.

Der pH-Wert stellt im Wesentlichen keinen limitierenden Parameter für das vorliegende Verfahren dar. Zur Vervollständigung der Hydrolyse des Cyanurchlorids kann es allerdings gemäß vorliegender Erfindung erforderlich sein, am Ende des Verfahrensschritts (d) den pH-Wert mit Hilfe einer Säure auf einen Wert zwischen 8,0 bis 9,5 einzustellen, was vorzugsweise mit einer Mineralsäure und dann bevorzugt mit Salzsäure geschieht. Ganz besonders bevorzugt ist im Verfahrensschritt (e) ein pH-Wert, der 8,5 bis 9,0 beträgt. Insgesamt empfiehlt sich ein erfindungsgemäßer Reaktionsablauf, bei dem das Reaktionsgemisch in den Reaktionsschritten (a) bis (d) einen bevorzugten pH-Wert ≥ 7,0 aufweist.

Im Verfahrensschritt (c), der Zugabe des organischen Alkalisalzes, kann verbrauchtes Na- bzw. KHCO₃ nachdosiert werden, wobei sich ein Überschuß an Alkalihydrogencarbonat aber erfahrungsgemäß überhaupt nicht nachteilig auf die Stabilität der AHDT-Lösung auswirkt, da sich das möglicherweise gebildete CO₂ in den entstehenden Natrium- oder Kalium-2-hydroxy-4,6-dichlor-s-triazin-Lösungen vollständig löst.

Gewisse Anforderungen an die nach dem vorliegenden Verfahren hergestellten AHDT-Lösungen können es ebenfalls erforderlich machen, die stabilisierte Lösung zusätzlich zu klären, wofür die Erfindung vorsieht, die Lösungen nach dem Reaktionsschritt d) oder e) zu filtrieren, was auch nach der Zugabe von Aktivkohle erfolgen kann.

Die so hergestellten stabilisierten wäßrigen Natrium- bzw. Kalium-2-hydroxy-4,6-dichlor-s-triazin-Lösungen werden bevorzugt zur Gelatinehärtung eingesetzt oder bei der Textil- oder Papierveredelung.

Insgesamt stellt die vorliegende Erfindung also ein Verfahren zur Verfügung, mit dem problemlos stabilisierte wäßrige AHDT-Lösungen erhalten werden. Dafür hat sich der Einsatz von Alkalihydrogencarbonaten in ihrer Funktion als Co-Stabilisator insofern als günstig erwiesen, als dadurch die gesamte Verfahrensdauer drastisch verkürzt wird. Außerdem kann ggf. auf den Einsatz von Aceton völlig verzichtet werden.

Die nachfolgenden Beispiele verdeutlichen diese Vorteile der Erfindung.

### Beispiele

### Beispiel 1

4500 kg einer wäßrigen 0,5 Gew.-%igen NaHCO₃-Lösung wurden bei 20 °C im Rührkessel vorgelegt. Der pH-Wert betrug > 8,5. Anschließend wurde die berechnete Menge Cyanurchlorid (450 kg = 9 Gew.-% bezogen auf die wäßrige Hydrogencarbonat-Lösung) zugegeben. Während der Cyanurchlorid-Zugabe wurde unter starker Kühlung mit der Dosierung einer 25 %igen NaOH begonnen (783 kg). Der pH-Wert betrug > 8. Während einer Nachreaktionszeit wurden 0,013 Gew.-% Natriumlaurylsulfat (0,75 kg) zugegeben; verbrauchtes NaHCO₃ wurde ersetzt. Durch das Einbringen von 0,5 Gew.-% Natriumtetraborat (28,6 kg) erhielt man eine stabilisierte Lösung, deren pH-Wert durch die Zugabe von Salzsäure auf 8,5 eingestellt wurde. Die so erhaltene Lösung wurde zur Entfernung vorhandener Trübungen bzw. Partikel abschließend filtriert.

| Lagerversuche bei Raumtemperatur: | | | | | | |
|---|---|---|---|---|---|---|
| Tage | 0 (Start) | 47 | 69 | 99 | 116 | 256 |
| pH-Wert | 8,5 | 7,8 | 7,7 | 7,6 | 8,0 | 8,0 |

Im Vergleich wird eine nicht mit Stabilisator versetzte Lösung schon nach 8 Wochen Lagerung bei Raumtemperatur sauer (pH = 1).

### Beispiel 2

4500 kg einer wäßrigen 0,5 Gew.-%igen NaHCO₃-Lösung wurden bei 20 °C im Rührkessel vorgelegt. Der pH-Wert betrug > 8,5. Anschließend wurde die berechnete Menge Cyanurchlorid (450 kg = 9 Gew.-% bezogen auf die wäßrige Hydrogencarbonat-Lösung) zugegeben. Während der Cyanurchlorid-Zugabe wurde unter starker Kühlung mit der Dosierung einer 25 %igen NaOH begonnen (783 kg). Der pH-Wert betrug > 8. Während einer Nachreaktionszeit wurden 0,013 Gew.-% Natriumlaurylsulfat (0,75 kg) zugegeben; verbrauchtes NaHCO₃ wurde ersetzt. Durch das Einbringen von 0,5 Gew.-% Dinatriumhydrogenphosphat (28,6 kg) erhielt man eine stabilisierte Lösung, deren pH-Wert durch die Zugabe von Salzsäure auf 8,5 eingestellt wurde. Die so erhaltene Lösung wurde zur Entfernung vorhandener Trübungen bzw. Partikel abschließend filtriert.

| Lagerversuche bei Raumtemperatur: | | | | | |
|---|---|---|---|---|---|
| Tage | 0 (Start) | 47 | 69 | 116 | 156 |
| pH-Wert | 8,5 | 7,0 | 7,6 | 8,0 | 8,0 |

Im Vergleich wird eine nicht mit Stabilisator versetzte Lösung schon nach 8 Wochen Lagerung bei Raumtemperatur sauer (pH = 1).

### Beispiel 3

1,75 I deionisiertes Wasser mit 2 Gew.-% bezogen auf die Gesamtlösung an Aceton wurden bei 18 °C in einem 4 I Vierhalskolben, ausgestattet mit Rührer, pH-Elektrode, Thermometer, Dosimatzuführung und Pulvertrichter, vorgelegt. Anschließend wurden 9,4 Gew.-% bezogen auf die wäßrige Acetonlösung an Cyanurchlorid (177 g) rasch zugegeben. Unter pH-Kontrolle wurde die Suspension ab der Cyanurchlorid-Zuführung mit 307,2 g einer 25 %igen NaOH versetzt. Die Temperatur der Reaktion wurde unter 25 °C gehalten. Nach etwa 6 Stunden waren 62 % der ber. NaOH-Menge zudosiert. Die anschließend 24 Stunden bei Raumtemperatur unter pH-Kontrolle (pH = 8,5) gerührte klare Lösung wurde anschließend auf pH = 9,0 eingestellt (99,9 % der ber. NaOH-Menge sind verbraucht) und mit 14,6 g NaHCO₃ (0,65 Gew.-%) versetzt, wobei der pH-Wert auf 8,1 sank. Durch Zugabe von 11,2 g Natriumtetraborat (0,5 Gew.-%) und Salzsäure wurde der pH-Wert auf 8,5 eingestellt. Die Reaktionslösung wurde mit 2 g Aktivkohle 2 Stunden gerührt. Zur Entfernung der Aktivkohle wurde die Lösung über eine Filternutsche filtriert. Man erhielt eine stabile Lösung.

**Lagerversuche bei Raumtemperatur:**

| Tage | 0 (Start) | 12 | 28 | 43 | 63 | 195 | 256 |
|---|---|---|---|---|---|---|---|
| pH-Wert | 8,5 | 8,3 | 8,2 | 8,5 | 8,8 | 8,1 | 8,0 |

Im Vergleich wird eine nicht mit Stabilisator versetzte Lösung schon nach 8 Wochen Lagerung bei Raumtemperatur sauer (pH = 1).

### Beispiel 4

1,75 I deionisiertes Wasser mit 2 Gew.-% bezogen auf die Gesamtlösung an Aceton wurden bei 18 °C in einem 4 I Vierhalskolben, ausgestattet mit Rührer, pH-Elektrode, Thermometer, Dosimatzuführung und Pulvertrichter, vorgelegt. Anschließend wurden 9,4 Gew.-% bezogen auf die wäßrige Acetonlösung an Cyanurchlorid (177 g) rasch zugegeben. Unter pH-Kontrolle wurde die Suspension ab der Cyanurchlorid-Zuführung mit 307,2 g einer 25 %igen NaOH versetzt. Die Temperatur der Reaktion wurde unter 25 °C gehalten. Nach etwa 6 Stunden waren 62 % der ber. NaOH-Menge zudosiert. Die anschließend 24 Stunden bei Raumtemperatur unter pH-Kontrolle (pH = 8,5) gerührte klare Lösung wurde anschließend auf pH = 9,0 eingestellt (99,9 % der ber. NaOH-Menge sind verbraucht) und mit 14,6 g NaHCO₃ (0,65 Gew.-%) versetzt, wobei der pH-Wert auf 8,1 sank und durch die Zugabe von Natronlauge auf 8,5 hochgestellt wurde. Durch Zugabe von 2,3 g Kaliumhexacyanoferrat (II) (0,1 Gew.-%) wurde die Lösung stabilisiert.

| Lagerversuche bei Raumtemperatur: | | | | | | | |
|---|---|---|---|---|---|---|---|
| Tage | 0 (Start) | 12 | 28 | 43 | 63 | 195 | 256 |
| pH-Wert | 8,5 | 8,3 | 7,5 | 7,8 | 8,2 | 8,0 | 8,0 |

Im Vergleich wird eine nicht mit Stabilisator versetzte Lösung schon nach 8 Wochen Lagerung bei Raumtemperatur sauer (pH = 1).

## Patentansprüche

1. Verfahren zur Herstellung einer stabilisierten wässrigen Alkalimetall-2-hydroxy-4,6-dichlor-s-triazin-Lösung,
**dadurch gekennzeichnet**,
das
(a) eine wässrige Alkalihydrogencarbonatlösung und gegebenenfalls Acetonlösung vorgelegt wird, dann
(b) Cyanurchlorid und eine Alkalilauge unter solchen Bedingungen zudosiert werden, dass eine Temperatur von 25 °C nicht überschritten und ein pH-Wert von 5,5 nicht unterschritten wird,
(c) danach ein Alkalisalz von C₁₀-C₂₀-Alkylsulfaten zugesetzt wird,
(d) abschließend ein Stabilisator, ausgewählt aus einem Alkaliborat und/oder einem Alkali(hydrogen)phosphat und/oder einem Alkalihexacyanoferrat (II), zugegeben wird und
(e) gegebenenfalls der pH-Wert mit Säure auf den gewünschten Wert eingestellt wird.

2. Verfahren nach Anspruch 1,
**dadurch gekennzeichnet,**
**dass** bei Temperaturen zwischen 5 und 40 °C
(a) eine wässrige 0,1 bis 1,0 Gew.-%ige Alkalihydrogencarbonatlösung und gegebenenfalls eine 0,5 bis 5 Gew.-%ige Acetonlösung vorgelegt wird, dann
(b) 8 bis 16 Gew.-% Cyanurchlorid bezogen auf die Lösung aus (a) zugesetzt werden, wobei der erhaltenen Suspension während der Cyanurchlorid-Zugabe bei Temperaturen von maximal 25 °C 1 Equivalent bezogen auf Cyanurchlorid einer 25 bis 50 Gew.-%igen Alkalilauge so zugegeben wird, dass der pH des Reaktionsgemisches einen Wert von 5,5 nicht unterschreitet,
(c) danach während einer Rührzeit von 2 bis 30 Stunden 0,1 bis 1,5 Gew.-‰ eines Alkalilaurylsulfats bezogen auf die Gesamtlösung zugesetzt werden,
(d) abschließend 0,3 bis 1,2 Gew.-% bezogen auf die Gesamtlösung eines Alkaliborats und/oder Dialkalihydrogenphosphats und/oder Kaliumhexacyanoferrat (II) zugegeben werden und
(e) gegebenenfalls der pH-Wert mit einer Säure auf 8 bis 9,5 eingestellt wird.

3. Verfahren nach Anspruch 1 oder 2,
**dadurch gekennzeichnet,**
**daß** die Temperatur zwischen 10 und 20°C liegt.

4. Verfahren nach einem der Ansprüche 1 bis 3,
**dadurch gekennzeichnet,**
**daß** als Alkalimetallionen Natriumionen oder Kaliumionen eingesetzt werden.

5. Verfahren nach einem der Ansprüche 1 bis 3,
**dadurch gekennzeichnet,**
**daß** das jeweils eingesetzte Alkalimetallion in allen Verfahrensschritten gleich ist.

6. Verfahren nach einem der Ansprüche 1 bis 5,
**dadurch gekennzeichnet,**
**daß** im Reaktionsschritt (a) eine wäßrige 0,5 bis 0,8%-ige Alkalihydrogencarbonat-Lösung und/oder eine wäßrige 1,0 bis 2,0%ige Acetonlösung vorgelegt wird.

7. Verfahren nach einem der Ansprüche 1 bis 6,
**dadurch gekennzeichnet,**
**daß** im Reaktionsschritt (b) 10 bis 12 Gew.-% Cyanurchlorid bezogen auf die Lösung aus (a) zugesetzt werden.

8. Verfahren nach einem der Ansprüche 1 bis 6,
**dadurch gekennzeichnet,**
**daß** die Rührzeit im Reaktionsschritt (c) 3 bis 6 Stunden beträgt.

9. Verfahren nach einem der Ansprüche 1 bis 8,
**dadurch gekennzeichnet,**
**daß** als Alkaliborat Na₂B₄O₇, K₂B₄O₇, deren jeweilige Penta- oder Decahydrate oder Mischungen davon eingesetzt werden.

10. Verfahren nach einem der Ansprüche 1 bis 9,
**dadurch gekennzeichnet,**
**daß** das Reaktionsgemisch in den Reaktionsschritten (a) bis (d) einen pH-Wert ≥ 7,0 aufweist.

11. Verfahren nach einem der Ansprüche 1 bis 10,
**dadurch gekennzeichnet,**
**daß** im Reaktionsschritt (e) eine Mineralsäure, vorzugsweise Salzsäure verwendet wird.

12. Verfahren nach enem der Ansprüche 1 bis 11,
**dadurch gekennzeichnet,**
**daß** der pH-Wert im Verfahrensschritt (e) auf 8,5 bis 9,0 eingestellt wird.

13. Verfahren nach einem der Ansprüche 1 bis 12,
**dadurch gekennzeichnet,**
**daß** im Verfahrensschritt (c) bei Zugabe des organischen Alkalisalzes verbrauchtes Alkalihydrogencarbonat nachdosiert wird.

14. Verfahren nach einem der Ansprüche 1 bis 13,
**dadurch gekennzeichnet,**
**daß** die stabilisierte Lösung nach dem Reaktionsschritt (d) oder (e) gegebenenfalls durch Zugabe von Aktivkohle filtriert wird.

## Claims

1. A process for the preparation of a stabilized aqueous alkali metal-2-hydroxy-4,6-dichloro-s-triazine solution,
**characterized in that**
(a) an aqueous alkali hydrogencarbonate and optionally acetone solution serves as starting solution, then
(b) cyanuric chloride and an alkali lye are added under such conditions that the temperature does not rise above 25 °C and the pH does not drop below 5.5,
(c) subsequently an alkali salt of C₁₀-C₂₀ alkyl sulfates is added,
(d) finally a stabilizer selected from an alkali borate and/or an alkali (hydrogen)phosphate and/or an alkali hexacyanoferrate (II) is added,and
(e) the pH is adjusted optionally to the desired value with acid.

2. The process of claim 1,
**characterized in that**
at temperatures between 5 and 40 °C
(a) an aqueous 0.1 to 1.0 wt.% alkali hydrogencarbonate solution and optionally a 0.5 to 5 wt.% acetone solution is provided as starting solution, to which
(b) 8 to 16 wt.% cyanuric chloride, expressed in terms of the solution from (a), is added, 1 equivalent of a 25 - 50 wt.% alkali lye - expressed in terms of cyanuric chloride - being added to the resultant suspension during the addition of cyanuric chloride at a temperature not exceeding 25 °C in such a manner that the pH of the reaction mixture does not drop below 5.5,
(c) thereafter, during a stirring period of 2 to 30 hours, 0.1 to 1.5 wt.% - expressed in terms of the overall solution - of an alkali lauryl sulfate is added,
(d) finally, 0.3 to 1.2 wt.% - expressed in terms of the overall solution - of an alkali borate and/or of a dialkali hydrogenphosphate and/or of potassium hexacyanoferrate (II) is added, and
(e) the pH is optionally adjusted to a value in the range from 8 to 9.5 with an acid.

3. The process of claim 1 or 2,
**characterized in that**
the temperature is between 10 and 20 °C

4. The process according to one of claims 1 to 3,
**characterized in that**
sodium or potassium ions are used as alkali metal ions.

5. The process according to one of claims 1 to 3,
**characterized in that**
the same alkali metal ion is used in all steps of the process.

6. The process according to one of claims 1 to 5,
**characterized in that**
an aqueous 0.5 to 0.8 % alkali hydrogencarbonate solution and/or an aqueous 1.0 to 2.0 % acetone solution is used as starting solution in step (a) of the reaction.

7. The process according to one of claims 1 to 6,
**characterized in that**
10 to 12 wt.% of cyanuric chloride, expressed in terms of the solution from (a), is added in reaction step (b).

8. The process according to one of claims 1 to 6,
**characterized in that**
the stirring time in step (c) of the reaction is 3 to 6 hours.

9. The process according to one of claims 1 to 8,
**characterized in that**
Na₂B₄O₇, K₂B₄O₇, their respective pentahydrates or decahydrates or mixtures thereof are used as alkali borate.

10. The process according to one of claims 1 to 9,
**characterized in that**
the reaction mixture in reaction steps (a) to (d) has a pH ≥ 7.0.

11. The process according to one of claims 1 to 10,
**characterized in that**
a mineral acid, preferably hydrochloric acid, is used in reaction step (e).

12. The process according to one of claims 1 to 11,
**characterized in that**
the pH is adjusted in reaction step (e) to a value in the range from 8.5 to 9.0.

13. The process according to one of claims 1 to 12,
**characterized in that**
in reaction step (c), where organic alkali salt is added, spent alkali hydrogencarbonate is replaced.

14. The process according to one of claims 1 to 13,
**characterized in that**
the stabilized solution is filtered following reaction step (d) or (e), optionally by addition of activated carbon.

## Revendications

1. Procédé de préparation d'une solution aqueuse stabilisée de métal alcalin 2-hydroxy-4,6-dichloro-S-triazine,
**caractérisé en ce que**
(a) on fournit une solution aqueuse d'hydrogénocarbonate alcalin et éventuellement une solution d'acétone, puis
(b) on ajoute du chlorure de cyanure et une lessive alcaline dans des conditions telles que l'on ne dépasse pas une température de 25°C et que l'on ne va pas en dessous d'un pH de 5,5,
(c) on ajoute ensuite un sel alcalin de sulfates d'alkyles en C₁₀ à C₂₀,
(d) pour finir, on ajoute un stabilisant choisi parmi un borate alcalin et/ou un phosphate (hydrogéno) alcalin et/ou un hexacyanoferrate (II) alcalin et,
(e) on ajuste éventuellement le pH avec de l'acide à la valeur souhaitée.

2. Procédé selon revendication 1,
**caractérisé en ce que**
à des températures entre 5 et 40°C
(a) on fournit une solution aqueuse de d'hydrogénocarbonate alcalin à 0,1 à 1,0% en poids et,éventuellement une solution d'acétone à 0,5 à 5% en poids, puis
(b) on ajoute 8 à 16% en poids de chlorure de cyanure par rapport à la solution de (a), sachant que l'on ajoute à la suspension obtenue, pendant l'addition de chlorure de cyanure et à des températures d'au maximum 25°C, 1 équivalent par rapport au chlorure de cyanure d'une lessive alcaline à 25 à 50% en poids, de manière à ce que le pH du mélange réactionnel ne soit pas inférieur à une valeur de 5,5,
(c) on ajoute ensuite, sous agitation de 2 à 30 heures, 0,1 à 1,5% en poids d'un laurylsulfate alcalin par rapport à la solution totale,
(d) pour finir, on ajoute 0,3 à 1,2% en poids par rapport à la solution totale d'un borate alcalin et/ou d'un hydrogénophosphate di-alcalin et/ou d'hexacyanoferrate (II) de potassium et
(e) on ajuste éventuellement le pH de 8 à 9,5 avec un acide.

3. Procédé selon revendication 1 ou 2,
**caractérisé en ce que**
la température est comprise entre 10 et 20°C.

4. Procédé selon l'une quelconque des revendications 1 à 3,
**caractérisé en ce que**
on utilise comme ions de métal alcalin des ions de sodium ou des ions de potassium.

5. Procédé selon l'une quelconque des revendications 1 à 3,
**caractérisé en ce que**
l'ion de métal alcalin utilisé est identique dans toutes les étapes du procédé.

6. Procédé selon l'une quelconque des revendications 1 à 5,
**caractérisé en ce que**
à l'étape (a) de la réaction on fournit une solution aqueuse d'hydrogénocarbonate alcalin à 0,5 à 0,8% et/ou une solution aqueuse d'acétone à 1,0 à 2,0%.

7. Procédé selon l'une quelconque des revendications 1 à 6,
**caractérisé en ce que**
à l'étape (b) de réaction on ajoute 10 à 12% en poids de chlorure de cyanure par rapport à la solution de (a).

8. Procédé selon l'une quelconque des revendications 1 à 6,
**caractérisé en ce que**
le temps d'agitation à l'étape de réaction (c) dure 3 à 6 heures.

9. Procédé selon l'une quelconque des revendications 1 à 8,
**caractérisé en ce que**
on met en oeuvre, en tant que borate alcalin du Na₂B₄O₇, du K₂B₄O₇, leurs penta- ou décahydrates ou des mélanges de ceux-ci.

10. Procédé selon l'une quelconque des revendications 1 à 9,
**caractérisé en ce que**
le mélange réactionnel présente, aux étapes (a) à (e) de la réaction un pH ≥ 7,0.

11. Procédé selon l'une quelconque des revendications 1 à 10,
**caractérisé en ce que**
à l'étape (e) de la réaction on utilise un acide minéral, de préférence de l'acide chlorhydrique.

12. Procédé selon l'une quelconque des revendications 1 à 11,
**caractérisé en ce que**
on ajuste le pH de 8,5 à 9,0 à l'étape (e) de la réaction.

13. Procédé selon l'une quelconque des revendications 1 à 12,
**caractérisé en ce que**
à l'étape (c) du procédé on ajoute de l'hydrogénocarbonate alcalin consommé lors de l'addition du sel alcalin organique.

14. Procédé selon l'une quelconque des revendications 1 à 13,
**caractérisé en ce que**
on filtre la solution stabilisée après l'étape (d) ou (e) de la réaction, éventuellement en ajoutant du charbon actif.
